# FASCICULE DE BREVET EUROPEEN

(11) **EP 3 999 896 B1**
(45) Date de publication et mention de la délivrance du brevet: **24.06.2026**
(21) Numéro de dépôt: 20753402.5
(22) Date de dépôt: 16.07.2020
(51) Int. Cl.: G02B 21/00, G02B 21/06, G02B 26/00, C12M 3/00, C12M 1/32

(54) **DISPOSITIF D'OBSERVATION D'UNE CELLULE OU D'UN ENSEMBLE DE CELLULES VIVANTES**
VORRICHTUNG ZUR BEOBACHTUNG EINER LEBENDEN ZELLE ODER EINES SATZES LEBENDER ZELLEN
DEVICE FOR OBSERVING A LIVING CELL OR A SET OF LIVING CELLS

(30) Priorité: 18.07.2019 FR 1908163
(43) Date de publication de la demande: 25.05.2022
(73) Titulaire: Université de Versailles Saint-Quentin-en-Yvelines, 78000 Versailles (FR); Centre Hospitalier Intercommunal de Poissy Saint-Germain, 78100 Saint Germain en Laye (FR); Centre Hospitalier Universitaire de Bordeaux, 33400 Talence (FR); Centre National de la Recherche Scientifique, 75016 Paris (FR); UNIVERSITE DE BORDEAUX, 33000 Bordeaux (FR)
(72) Inventeur: VIALARD, François, 75007 PARIS (FR); JIMENEZ, Clément, 75016 PARIS (FR); GEINDRE, Christophe, 33600 PESSAC (FR); HIBERTY, Bruno, 33600 PESSAC (FR); DUCROS, Hugo, 33600 PESSAC (FR)
(74) Mandataire: Novagraaf Technologies
(86) Numéro de dépôt international: PCT/FR2020/051277
(87) Numéro de publication internationale: WO 2021/009465

(56) Documents cités:
- WO-A1-2016/145476
- WO-A1-2019/141951
- US-A- 4 852 985
- US-A1- 2009 133 170
- US-A1- 2016 187 359
- US-B1- 6 238 911
- "Atlas of Time Lapse Embryology", 21 January 2015, CRC PRESS, article ALISON CAMPBELL ET AL: "Atlas of Time Lapse Embryology", XP055695486
- VITROLIFE: "Preparation of EmbryoSlide Flex culture dishes", 1 May 2019 (2019-05-01), XP055695962, Retrieved from the Internet <URL:https://www.vitrolife.com/globalassets/support-documents/tech-notes/technote_embryoscope-flex-dish-preparation_v1.pdf> [retrieved on 20200515]
- RYO SUGIMOTO ET AL: "Contrast enhancement by oblique illumination microscopy with an LED array", OPTIK., vol. 183, 1 April 2019 (2019-04-01), DE, pages 92 - 98, XP055741405, ISSN: 0030-4026, DOI: 10.1016/j.ijleo.2019.02.068

## Description

### [Domaine technique

La présente invention se rapporte à un dispositif d'imagerie pour l'observation d'une cellule ou d'un ensemble de cellules vivantes dans le cadre de l'étude de la reproduction et en particulier pour la Fécondation *In Vitro* (FIV).

Plus précisément, l'invention concerne un dispositif d'imagerie pour l'observation d'une cellule ou d'un ensemble de cellules vivantes, une boîte de Petri associée ainsi qu'un procédé et un produit programme d'ordinateur adaptés à cette observation.

### Etat de la technique

Les dispositifs d'imagerie actuels pour l'observation d'une cellule ou d'un ensemble de cellules vivantes tel que des embryons sont en majorité des microscopes équipés d'objectifs de grossissement différents, d'un système d'observation direct monoculaire ou binoculaire et généralement d'un système d'observation déporté pour permettre la visualisation de la cellule ou de l'ensemble de cellules vivantes sur un écran vidéo.

La cellule ou l'ensemble de cellules vivantes sont placés sur un support puis sont observés à l'aide de différents objectifs permettant d'agrandir ou de réduire la taille des images obtenues et ainsi de visualiser de la cellule ou l'ensemble de cellules vivantes selon différents grossissements et donc dans le détail ou de manière globale.

Ces dispositifs ne sont pas très précis dans la manipulation, ni faciles d'utilisation. Il faut commencer par regarder la cellule ou l'ensemble de cellules vivantes avec un faible grossissement pour le localiser et le centrer dans le champ du microscope en le déplaçant manuellement puis changer d'objectif en utilisant des grossissements de plus en plus important pour regarder la cellule ou l'ensemble de cellules vivantes dans le détail.

Dans l'exemple particulier de l'observation du développement d'embryons et de la réalisation d'une Fécondation *In Vitro* (FIV), les embryons destinés à être implantés sont sélectionnés en fonction de leur qualité cellulaire, à savoir : leur nombre de cellules, leur régularité (tailles des cellules différentes ou non), et de leur fragmentation. Les embryons sélectionnés sont, en priorité, ceux dont la chronologie de la division cellulaire est respectée, avec des cellules bien régulières et sans fragmentation. Ces paramètres sont censés être un indicateur des meilleures chances de grossesse.

Cette observation se fait en général hors des enceintes de culture embryonnaire adaptées pour reconstituer un environnement idéal, en température et en concentration de gaz, pour le bon développement des embryons. Le document US 2015/0278625 A1 décrit un tel mode d'observation hors incubateur. Or, il a été démontré qu'une observation hors des enceintes et surtout le changement brutal d'environnement pour les embryons joue un rôle potentiellement délétère sur le développement de ceux-ci.

Des dispositifs d'imagerie d'embryons ont donc été développés dans le cadre des techniques de Fécondations In Vitro (FIV) afin de permettre une observation in vitro du développement des embryons, après fécondation, dans ce type d'environnement idéal à leur développement.

Cette observation peut se poursuivre sur 2 à 5 jours en fonction des centres de FIV et permet de mieux sélectionner les embryons qui seront transférés dans les voies génitales de la patiente et donc d'améliorer les taux de succès de la FIV.

Deux catégories de dispositifs d'observation existent soit le dispositif d'observation est directement intégré dans un système adapté pour reconstituer l'environnement idéal pour le bon développement des embryons, avec des chambres de culture individuelles comprises recevant des boîtes spécifiques à chaque dispositif ; soit le dispositif d'observation est individuel et à mettre dans des enceintes préexistantes avec des boîtes également spécifiques pour l'observation.

Toutes ces spécificités rendent les dispositifs actuels ainsi que leur utilisation très coûteux limitant ainsi leur accès aux structures médicales au budget restreint ou nécessitant une augmentation des frais et/ou des coûts de prise en charge pour les bénéficiaires d'une FIV.

Dans tous les cas, que ce soit dans le cas de cellules vivantes ou ensembles de cellules vivantes, ou dans le cas particulier des embryons, la qualité optique des images, l'identification précise des cellules vivantes ou ensembles de cellules vivantes et le suivi temporel et régulier de leur développement sont des paramètres essentiels à l'utilisation de ces procédés. Or, d'une part, les systèmes actuels ne permettent pas une localisation précise des cellules vivantes ou ensembles de cellules vivantes, ceux-ci étant confinés dans les espaces réduits limitant le champ d'observation dans les 3 axes x, y et z. Dans le cas d'un embryon, ses mouvements naturels peuvent le faire sortir du champ de la zone observable sans possibilité d'une relocalisation ou le faire apparaître en bordure de champ, là où les aberrations optiques sont les plus importantes ; d'autre part, l'observation sous une seule incidence n'a pas permis jusqu'à maintenant de pouvoir créer des algorithmes susceptibles de réaliser une aide à l'interprétation.

Un autre paramètre important est le temps d'observation des cellules vivantes ou ensembles de cellules vivantes, lesquels constituant des échantillons, qui impacte l'efficacité des analyses effectuées sur ceux-ci. Les dispositifs actuels disposent en général de moyens de déplacement mécaniques selon 3 axes communément appelés x, y (définissant un plan horizontal où sont contenus les échantillons à observer) et z (direction perpendiculaire au plan précédent), dans la direction de la profondeur des échantillons) afin de positionner de manière optimale les échantillons. Ces moyens de déplacement mécaniques réalisent des déplacements pouvant être coûteux en temps. Une solution dans l'état de la technique pour réduire le temps de déplacement en profondeur est d'utiliser une lentille liquide, dont la distance focale peut être variée électroniquement, comme dans les demandes de brevet relatives au domaine dentaire US2014/0002626 A1, traitant d'une caméra intra-buccale autofocus, et EP 2 161 607 A1, traitant d'une caméra dentaire.

Un exemple de dispositif d'imagerie pour l'observation de cellules vivantes est divulgué dans le document US2016/187359A1. Le document US 4 852 985 A décrit un système d'éclairage pour un microscope.

Il existe donc un réel besoin d'un dispositif d'imagerie palliant les défauts, inconvénients et obstacles de l'art antérieur, en particulier d'un dispositif et d'un procédé permettant d'améliorer la qualité optique et l'efficacité temporelle de l'observation de cellules vivantes ou d'ensembles de cellules vivantes, tout en réduisant les coûts de fabrication et d'utilisation du dispositif. Dans le cas particulier de la FIV, on recherche une meilleure accessibilité de celle-ci, par une réduction de coût, aux personnes concernées.

### Description de l'invention

Pour résoudre un ou plusieurs des inconvénients cités précédemment, le demandeur propose un dispositif d'imagerie selon la revendication 1; en particulier un dispositif d'imagerie pour l'observation du développement de cellules vivantes ou d'ensembles de cellules vivantes (80) dans le cadre de l'étude de la reproduction et en particulier pour la Fécondation In Vitro (FIV), caractérisé en ce qu'il comprend :
- un système d'imagerie comprenant une caméra grand champ adaptée à l'identification l'observation d'une ou plusieurs cellules vivantes ou ensembles de cellules vivantes à observer, les cellules vivantes ou ensembles de cellules vivantes étant déposés dans une boîte de Petri contenant un logement spécifique pour chaque cellule vivante ou ensemble de cellules vivantes ;
- un support apte à recevoir la boîte de Petri et positionné entre le système d'éclairage et le système d'imagerie ;
- un système d'éclairage comprenant trois sources lumineuses, adaptées pour éclairer un objet qui est une cellule vivante ou un ensemble de cellules vivantes à observer, dont une source centrale, une première source latérale placée d'un première côté de la source centrale, et une deuxième source latérale placée d'un deuxième côté de la source centrale,

le système d'éclairage étant configuré pour mettre en œuvre chacun des types d'éclairage particuliers suivants :
   - un éclairage de type « repérage » dans lequel ladite source lumineuse (111) est configurée pour permettre permettant de repérer la ou les cellules vivantes ou ensembles de cellules vivantes à observer par la caméra grand champ du système d'imagerie, ledit éclairage de type « repérage » étant réalisé en mettant en œuvre la source centrale, la première source latérale et la deuxième source latérale, les rayons lumineux issus des sources lumineuses étant focalisés sur l'objet selon un cône ayant une ouverture angulaire comprise entre 26° et 34° ;
   - un éclairage de type « contours » dans lequel ladite source lumineuse (111) est configurée pour permettre permettant de compter la ou les cellules présentes dans la cellule vivante ou l'ensemble de cellules vivantes observé par la caméra grand champ du système d'imagerie,
ledit éclairage de type « contours » étant réalisé en mettant en œuvre la première source latérale et la deuxième source latérale, les rayons issus de la première source latérale et de la deuxième source latérale étant séparés en deux faisceaux collimatés symétriques par rapport à l'axe perpendiculaire au plan formé par le support de la boîte de Petri ou la boîte de Petri elle-même, un premier faisceau issu de la première source latérale éclairant l'objet selon un angle d'incidence par rapport à l'axe perpendiculaire comprise entre 10° et 14°, un deuxième faisceau issu de la deuxième source latérale éclairant l'objet selon un angle d'incidence par rapport à l'axe perpendiculaire comprise entre 10° et 14° ;
   - un éclairage de type « relief » dans lequel ladite source lumineuse (111) est configurée pour permettre permettant de visualiser la texture et la granularité de la ou des cellules présentes dans la cellule vivante ou l'ensemble de cellules vivantes observé par la caméra grand champ du système d'imagerie, ledit éclairage de type « relief » étant réalisé en mettant en œuvre la première source latérale, un unique faisceau lumineux collimaté issu de la première source latérale se propageant selon un axe incliné d'un angle par rapport à l'axe perpendiculaire compris entre 8° et 16°;
   - des moyens de déplacement relatif de la boîte de Petri par rapport à l'ensemble formé par le système d'éclairage et ladite caméra grand champ de sorte à pouvoir observer des cellules vivantes ou ensembles de cellules vivantes situés dans les différents logements de la boîte de Petri,
ladite caméra grand champ présentant un champ de vue couvrant au moins la surface totale d'un des logements de la boîte de Petri, et ledit dispositif d'imagerie étant adapté pour imager, sans déplacement relatif de ladite boîte de Petri avec les moyens de déplacement par rapport à l'ensemble formé par le système d'éclairage et ladite caméra grand champ, une cellule vivante ou un ensemble de cellules vivantes dans une position quelconque dans son logement dans la boîte de Petri et ladite caméra grand champ présentant une résolution adaptée à l'observation des détails d'une cellule vivante ou d'un ensemble de cellules vivantes, lesdits détails ayant une taille micrométrique

Le système d'éclairage spécifique du dispositif selon l'invention permet de rendre le dispositif compact , multifonction, et ne nécessitant aucun démontage de pièce pour passer d'un type d'éclairage à l'autre. Les manipulations sont réduites au strict nécessaire, la simplicité d'utilisation, dans la durée, directement dans l'incubateur , sans risque de perturbations pour l'embryon, offre des conditions idéales d'observation.

L'éclairage de type « contours » permet de compter facilement le nombre de cellules, en une seule vue, y compris quand les cellules sont empilées.

L'éclairage de type « relief » donne des informations sur la texture de l'enveloppe des cellules. Cet éclairage est présent en deux directions opposées (haut/bas) donnant des informations complémentaires pouvant être utiles à la compréhension de la géométrie de l'embryon, formant ainsi la troisième combinaison.

Le champ de vue de la caméra grand champ, couvrant au moins la surface totale d'un des logements de la boîte de Petri, permet ainsi d'imager directement, sans utiliser de moyen de déplacement, une cellule vivante ou un ensemble de cellules vivantes positionné dans une position quelconque dans son logement dans la boîte de Petri.

Par « caméra grand champ » au sens de l'invention, on entend notamment une caméra présentant un champ de vision supérieure à 1 mm², avec une résolution pixel inférieure à 1µm.

De préférence, les cellules vivantes ou ensembles de cellules vivantes à observer sont un ou plusieurs embryons.

Selon une variante, les moyens de déplacement peuvent être aptes à déplacer l'ensemble formé par le système d'éclairage et la caméra grand champ du système d'imagerie relativement par rapport à la boîte de Petri dans laquelle sont déposés les cellules vivantes ou ensembles de cellules vivantes à observer.

Dans un mode de réalisation, le dispositif d'imagerie selon l'invention peut comprendre en outre une lentille liquide, adaptée pour contrôler une distance de focalisation dans la direction de la profondeur de la cellule vivante ou de l'ensemble de cellules vivantes.

Un autre objet de la présente demande porte sur un procédé d'observation selon la revendication 5, notamment un procédé d'observation du développement de cellules vivantes ou ensembles de cellules vivantes par un dispositif d'imagerie selon l'invention, comprenant les étapes suivants :
- une étape de préparation consistant à déposer successivement une cellule vivante ou un ensemble de cellules vivantes dans une boîte de Petri,
la boîte de Petri comprenant :
- un récipient adapté pour recevoir une ou plusieurs cellules vivantes ou ensembles de cellules vivantes à observer,
- un couvercle,
- un élément d'identification adapté à l'identification de la ou des cellules vivantes ou ensembles de cellules vivantes observés,
- des logements ou puits spécifiques destinés à la dépose d'une cellule vivante ou d'un ensemble de cellules vivantes, lesdits logements ou puits étant réalisés sous forme de cupules adaptées pour recevoir, en plus de la cellule vivante ou l'ensemble de cellules vivantes dans la cupule, une goutte d'un milieu de culture, puis une goutte d'un milieu de culture dans ladite boîte de Petri, l'opération étant réitérée autant de fois que nécessaire en fonction du nombre de cellules vivantes ou ensembles de cellules vivantes à observer souhaité ; puis à recouvrir le tout avec un liquide, tel que de l'huile ou de l'eau;
- une étape d'identification de la boîte de Petri consistant à détecter et visualiser, à l'aide d'un lecteur dédié, l'élément d'identification de la boîte de Petri dans laquelle se trouve la ou les cellules vivantes ou ensembles de cellules vivantes à observer ; et
- une étape d'observation d'une première cellule vivante ou ensemble de cellules vivantes à l'aide du système d'imagerie.

Avantageusement, une étape optionnelle, préalable à l'étape d'identification de la boîte de Petri, de positionnement approximatif de la boîte de Petri par rapport à l'ensemble formé par le système d'éclairage et la caméra grand champ du système d'imagerie relativement à la boîte de Petri à l'aide des moyens de déplacement est réalisée, de sorte que la boîte de Petri soit approximativement alignée avec l'ensemble formé par le système d'éclairage et la caméra grand champ du système d'imagerie ;

Avantageusement, le procédé d'observation du développement de cellules vivantes ou ensembles de cellules vivantes peut comprendre, après l'étape d'observation d'une première cellule vivante ou d'un premier ensemble de cellules vivantes , une étape de déplacement relatif de la boîte de Petri par rapport à l'ensemble formé par le système d'éclairage et la caméra grand champ du système d'imagerie permettant l'observation d'une autre cellule vivante ou d'un autre ensemble de cellules vivantes situé dans un autre logement de la boîte de Petri.

Dans le mode de réalisation où le dispositif d'imagerie selon l'invention comprend en outre une lentille liquide, l'étape d'observation d'une première cellule vivante ou d'un premier ensemble de cellules vivantes dans le procédé d'observation du développement de cellules vivantes ou ensembles de cellules vivantes peut être effectuée dans un plan d'observation horizontal parallèle au support apte à recevoir la boîte de Petri et perpendiculaire à la direction de la profondeur de ladite première cellule vivante ou dudit ensemble de cellules vivantes , ledit plan étant déterminé dans une étape supplémentaire de détermination par une configuration de la lentille liquide.

Avantageusement, dans le même mode de réalisation où le dispositif d'imagerie selon l'invention comprend en outre une lentille liquide, l'étape de détermination d'un plan d'observation de ladite première cellule vivante ou dudit ensemble de cellules vivantes est répétée pour différents plans perpendiculaires à la direction de la profondeur de ladite première cellule vivante ou dudit ensemble de cellules vivantes par différentes configurations de la lentille liquide, de sorte à observer une première cellule vivante ou d'un premier ensemble de cellules vivantes dans les différents plans d'observation déterminés.

L'observation en différents plans perpendiculaires à la direction de la profondeur de ladite première cellule vivante ou dudit ensemble de cellules vivantes permise par la présence d'une lentille liquide dans le dispositif d'imagerie selon l'invention permet ainsi de s'affranchir d'un déplacement dans la direction de la profondeur de ladite première cellule vivante ou dudit ensemble de cellules vivantes, et ainsi un gain de temps dans la procédure d'analyse et d'observation de la/des cellules vivantes ou ensembles de cellules vivantes.

D'autres avantages et particularités de la présente invention résulteront de la description qui va suivre, donnée à titre d'exemple non limitatif et faite en référence aux figures annexées :
- La figure 1 représente un premier mode de réalisation d'un dispositif d'imagerie selon l'invention ;
- La figure 2 représente un mode de réalisation d'un éclairage de type « repérage » ;
- La figure 3 représente un mode de réalisation d'un éclairage de type « contours » ;
- La figure 4 représente un mode de réalisation d'un éclairage de type « texture » ;
- La figure 5 représente un deuxième mode de réalisation d'un dispositif d'imagerie selon l'invention ;
- La figure 6 représente un dispositif d'imagerie selon un autre mode de réalisation de l'invention disposé à l'intérieur d'un incubateur ;
- La figure 7a représente une vue de profil du dispositif d'imagerie disposé à l'intérieur d'un incubateur de la figure 6 ;
- La figure 7b représente une vue du dessus du dispositif d'imagerie disposé à l'intérieur d'un incubateur de la figure 6 ;
- La figure 8a représente plusieurs vues bidimensionnelles (dessus, côté, coupe transversale) d'un exemple de boîte de Petri pouvant être utilisée dans l'invention ;
- La figure 8b représente une vue 3D de l'exemple de boîte de Petri de la figure 8a ;
- La figure 9a représente une vue de profil d'un mode de réalisation d'une boîte de Petri, selon l'invention, posée sur un support ;
- La figure 9b représente une vue de dessus de la boîte de Petri de la figure 9a posée sur un support ;
- La figure 10 représente un réservoir à embryon présent sur le fond d'une boîte de Petri selon un mode de réalisation de l'invention ;
- Les figures 11a à 11c représentent un autre mode de réalisation d'une boîte de Petri selon l'invention, respectivement la figure 11a représente une vue de profil, la figure 11b une vue du dessus et la figure 11c une coupe du profil de la boîte de Petri ;
- La figure 12 représente le synoptique d'un procédé d'observation du développement d'une cellule vivante ou ensemble de cellules vivantes selon un premier mode de réalisation de l'invention ;
- La figure 13 représente une image plein champ obtenue par le premier mode de réalisation du procédé d'observation selon l'invention avec la caméra grand champ du système d'imagerie du dispositif d'imagerie selon l'invention ;
- La figure 14 représente le synoptique d'un procédé d'observation du développement d'une cellule vivante ou ensemble de cellules vivantes selon un deuxième mode de réalisation de l'invention ;
- La figure 15 représente le synoptique d'un procédé d'observation du développement d'une cellule vivante ou ensemble de cellules vivantes selon un troisième mode de réalisation de l'invention;
- La figure 16 montrent deux images d'un embryon obtenu avec un dispositif d'imagerie et selon le premier mode de réalisation du procédé d'observation selon l'invention, avec deux types d'éclairage différents, respectivement un éclairage de type « contours » à fauche et un éclairage de type « texture » à droite ;
- La figure 17a représente une succession de coupes transversales d'un embryon dans sa profondeur obtenu avec un dispositif selon l'invention et selon le troisième mode de réalisation du procédé d'observation selon l'invention, en utilisant un éclairage de type « contours » ;
- La figure 17b représente une succession de coupes transversales d'un embryon dans sa profondeur obtenu avec un dispositif selon l'invention et selon le troisième mode de réalisation du procédé d'observation selon l'invention, en utilisant un éclairage de type « texture ».

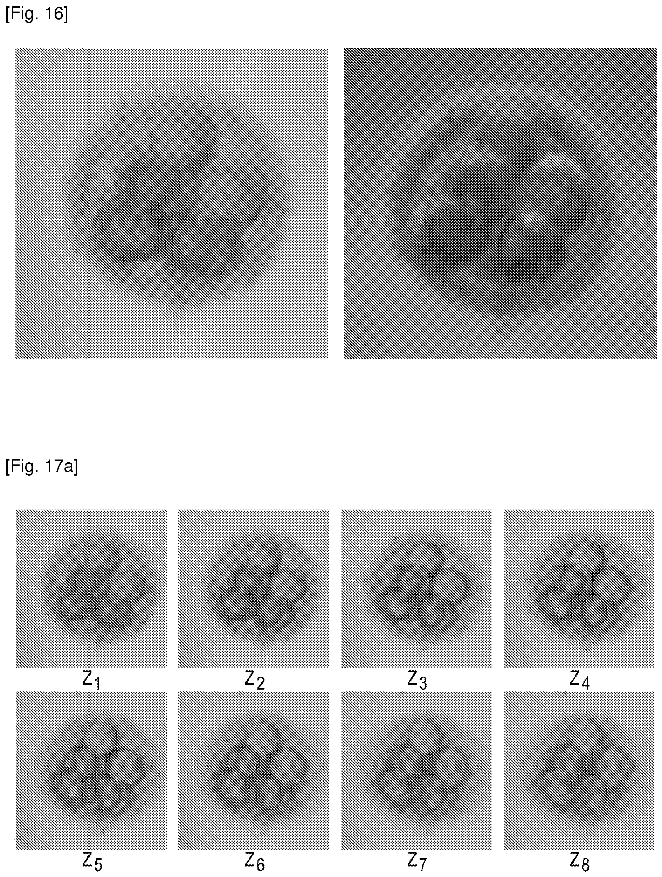

Les figures 16 à 17b sont commentées plus en détail au niveau des exemples qui suivent, qui illustrent l'invention sans en limiter la portée. Les autres figures, décrivant différents modes de réalisation du dispositif, de la boîte de Petri et du procédé d'observation selon l'invention et utilisés ou mis en œuvre dans les exemples, sont détaillées ci-après.

### DISPOSITIF D'IMAGERIE SELON L'INVENTION

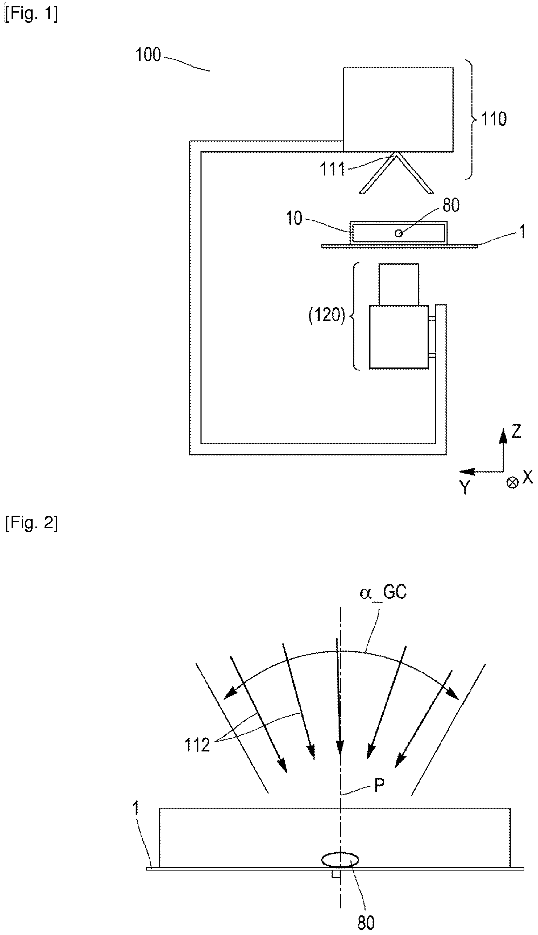

On réalise un dispositif d'imagerie 100 selon un premier mode de réalisation de l'invention tel qu'illustré sur la figure 1, à partir des éléments suivants :
- système d'éclairage 110 : éclairage de type LED (diodes électroluminescentes)
- système d'imagerie 120 comprenant une caméra grand champ 121 équipée d'un capteur d'image présentant une résolution de l'ordre de 5 pixels par 1 micromètre observé
- un support 1 : une vitre
- des moyens de déplacement relatif de type platines motorisées.

Ces éléments sont indiqués à titre d'exemple, et le dispositif selon l'invention peut être réalisé avec des éléments présentant des paramètres différents.

Le dispositif d'imagerie 100 réalisé est destiné à observer une cellule vivante ou un ensemble de cellules vivantes 80 contenu dans une boîte de Petri 10 posée sur le support 1. Par le terme « observer », il est entendu le fait de détecter et de distinguer les détails d'une structure.

Dans le mode de réalisation présenté à la figure 1, le dispositif 100 est réalisé en configuration « microscope inversé », c'est-à-dire que la cellule ou l'ensemble de cellules vivantes observé 80 est éclairé par le dessus et que le système d'imagerie 120 se trouve en dessous et observe la lumière transmise par la cellule vivante ou l'ensemble de cellules vivantes 80 qui est un objet translucide. Pour la suite de la description, c'est cette configuration qui est retenue, cependant le dispositif 100 peut aussi bien être réalisé en configuration « normale » (la cellule ou l'ensemble de cellules vivantes 80 éclairé par le dessous et observé vu de dessus comme avec un microscope de base), qu'en configuration « inversée ».

La prise d'image par la caméra grand champ 121 se fait en transmission à travers la cellule ou l'ensemble de cellules vivantes 80 soit par la technique dite de diascopie. C'est-à-dire que l'éclairage se trouve d'un côté de l'objet à imager et la caméra de l'autre côté de l'objet.

[37] Ainsi la boîte de Petri 10 dans laquelle se trouve la ou les cellules vivantes ou ensembles de cellules vivantes 80 à observer doit être transparente à la lumière utilisée pour éclairer la ou les cellules vivantes ou ensembles de cellules vivantes 80. La boîte de Petri 10 est posée sur le support 1, comme dans le mode réalisation présenté à la figure 1. Ce support 1 doit aussi être transparent à la lumière utilisée pour éclairer la cellule vivante ou ensemble de cellules vivantes 80, comme une vitre en verre pour les microscopes classiques dont la lumière est une ampoule émettant dans le visible. Dans le dispositif présenté ici, ce support 1 est une vitre mais peut être encore un tiroir coulissant sur lequel peut être déposé une ou plusieurs boîtes de Petri 10.

Avantageusement, ce support 1 peut comprendre des plots ou rebord permettant de pré-positionner la ou les boîtes de Petri 10 sur des positions prédéfinies. Dans ce cas, aucun réglage latéral préliminaire, c'est-à-dire, dans un plan parallèle au plan défini par le support 1, n'est utile pour observer une cellule vivante ou un ensemble de cellules vivantes 80 grâce au large champ de vue de la caméra grand champ 121 qui, couvrant au moins l'intégralité d'un logement 61, appelé encore puits ou cupule, d'une boîte de Petri 10 permet de repérer directement une cellule vivante ou un ensemble de cellules vivantes 80 quelle que soit la position de celle-ci ou de celui-ci dans son logement 61.

Avantageusement, le système d'éclairage 110 et la caméra grand champ 121 sont solidaires et montés sur une structure en forme de « U » de sorte que le système d'éclairage 110 se trouve en face de la caméra grand champ 121 comme sur la Figure 1.

Avantageusement, le système d'éclairage 110 est choisi parmi au moins l'un des types d'éclairage particuliers suivants :
- l'éclairage de type « repérage » dans lequel la au moins une source lumineuse 111 est configurée pour permettre de repérer la ou les cellules vivantes ou ensembles de cellules vivantes 80 à observer par le système d'imagerie 120 ;
- l'éclairage de type « contours » dans lequel la au moins une source lumineuse 111 est configurée pour permettre de compter la ou les cellules présentes dans la cellule vivante ou l'ensemble de cellules vivantes 80 observé par le système d'imagerie 120 ; et
- l'éclairage de type « relief » dans lequel la au moins une source lumineuse 111 est configurée pour permettre de visualiser la texture et la granularité de la ou des cellules présentes dans la cellule vivante ou l'ensemble de cellules vivantes 80 observé par le système d'imagerie 120.

L'éclairage de type « repérage », est de préférence utilisé pour la détection et la visualisation, par le système d'imagerie 120, pour l'imagerie et l'analyse d'un élément d'identification 50 présent sur la boîte de Petri 10 et permettant l'identification de la ou des cellules vivantes ou l'ensemble ou les ensembles de cellules vivantes 80 à observer. Ainsi le choix de la au moins une source lumineuse 111 et de la caméra grand champ 121 doit être configuré. Par exemple, la sensibilité de la caméra grand champ 121 doit être optimisée en fonction de la longueur d'onde émise par la source lumineuse 111.

Cet éclairage de type « repérage » peut aussi être utilisé pour aider à la détection de la cellule vivante ou l'ensemble de cellules vivantes 80 par la caméra grand champ 121 et système d'imagerie 120.

La figure 2 représente un mode de réalisation d'un éclairage de type « repérage ». Dans cette figure 2, l'objet à identifier ou détecter à l'aide de la caméra grand champ 121 et du système d'imagerie 120, qui peut être l'élément d'identification 50 de la boîte de Petri ou comme dans la figure 2 la cellule vivante ou l'ensemble de cellules vivantes 80, est éclairé par un éclairage de type homogène ou diffus. Les rayons lumineux 112 issus de la source lumineuse 110 sont concentrés ou focalisés sur l'objet à repérer ou détecter, dans un cône ayant une ouverture angulaire α_GC de l'ordre de 30°. L'objet observé (élément d'identification 50 ou cellule vivante ou ensemble de cellules vivantes 80) est donc éclairé selon des angles d'incidence variant entre -15° et +15° par rapport à l'axe perpendiculaire P au plan formé par le support 1 de la boîte de Petri 10 ou la boîte de Petri 10 elle-même.

Le cône d'ouverture angulaire α_GC est de l'ordre de 30° ± 4°. Les angles d'incidence varient entre -15° ± 2° et + 15° ± 2°.

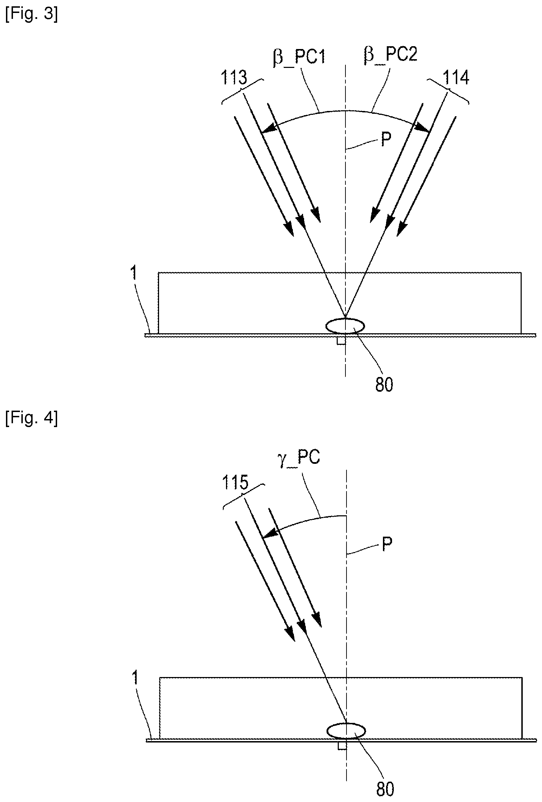

La figure 3 représente un mode de réalisation d'un éclairage de type « contours ». Dans cette figure 3, la cellule vivante ou ensemble de cellules vivantes 80 à observer par la caméra grand champ 121 peut être éclairée selon deux axes de préférence symétriques par rapport à l'axe perpendiculaire P au plan formé par le support 1 de la boîte de Petri 10 ou la boîte de Petri 10 elle-même. Ainsi, les rayons issus de la au moins une source lumineuse 111 sont séparés en deux faisceaux collimatés 113 et 114. Le premier faisceau 113 éclaire l'embryon 80 selon un angle d'incidence β_PC1 par rapport à l'axe perpendiculaire P et le second faisceau 114 éclaire l'embryon 80 selon un angle d'incidence β_PC2. Comme énoncé précédemment, les angles β_PC1 et β_PC2 sont de préférence égaux et opposés (i.e. β_PC1 = -β_PC2), c'est-à-dire que les faisceaux 113 et 114 sont de préférence symétriques par rapport à l'axe perpendiculaire P. Typiquement, les angles β_PC1 et β_PC2 sont de l'ordre de +/- 12° ± 2°. L'angle total entre les deux directions formées par les faisceaux 113 et 114 est donc typiquement de l'ordre de 24° ± 4°. La haute résolution de la caméra grand champ 121 (permettant d'observer les détails d'une cellule vivante ou d'un ensemble de cellules vivantes 80, lesdits détails ayant une taille micrométrique) coopère avec cet éclairage de type « contours ».

L'éclairage de type « texture » est de préférence utilisé pour optimiser la visualisation, par le système d'imagerie 120, de la texture et la granularité des cellules présentes dans la cellule vivante ou l'ensemble de cellules vivantes 80 observé de sorte à permettre d'apprécier son relief et sa profondeur.

La figure 4 représente un mode de réalisation d'un éclairage de type « texture ». Dans cette figure 4, la cellule vivante ou l'ensemble de cellules vivantes 80 à observer par la caméra grand champ 121 et dont la texture et la granularité des cellules vont être visualisées, peut être éclairé par un seul faisceau lumineux 115. Ce faisceau 115 est collimaté et se propage selon un axe incliné d'un angle y_PC par rapport à l'axe perpendiculaire P. La valeur absolue de cet angle γ_PC varie typiquement entre 8° et 16°. Aucune direction d'incidence n'est privilégiée par rapport à l'axe perpendiculaire P ou la cellule vivante ou l'ensemble de cellules vivantes 80.

Pour faciliter la réalisation de ces différents éclairages, le système d'éclairage 110 peut être composé d'au moins une ou plusieurs sources lumineuses 111, de type DEL (ou LED en anglais). Chacune de ces sources lumineuses 111 peut être commandée individuellement. Il est à noter que les LED ont pour avantage d'être très peu volumineuses de sorte qu'il est possible de dupliquer l'éclairage par un ensemble de LEDs de sorte qu'une LED éclaire une cellule vivante ou ensemble de cellules vivantes 80.

Dans le cas particulier où l'ensemble de cellules vivantes à observer est un embryon, la longueur d'onde de la au moins une source lumineuse 111 utilisée (LED ou autre) peut se situer, de préférence, dans le domaine du rouge (aux alentours de 630 nm), qui est le domaine le moins nocif pour l'embryon 80. De plus, il est préférable que la source lumineuse 111 soit commandée en mode pulsé de sorte à limiter la durée cumulée d'exposition de l'embryon 80 à la lumière à quelques dizaines de secondes par jour, cela pour ne pas l'endommager.

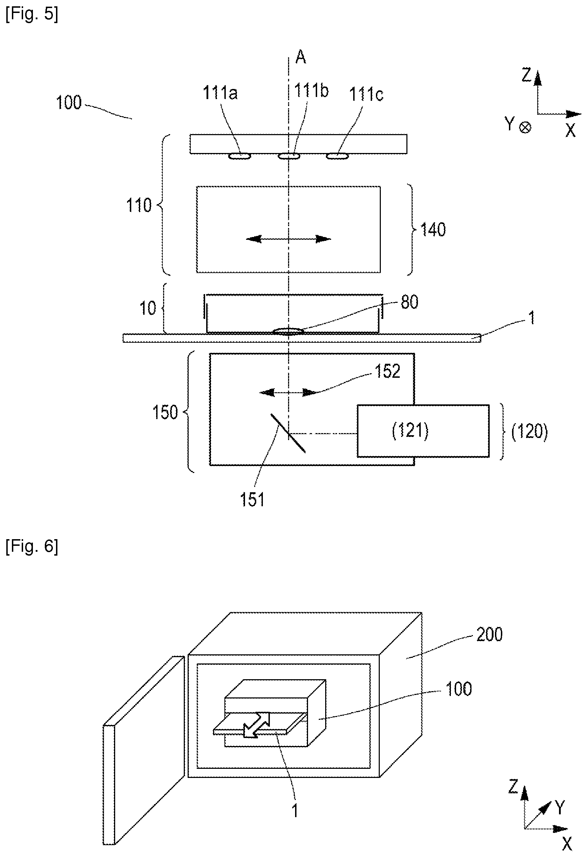

La figure 5 illustre un mode de réalisation de l'invention où le système d'éclairage 110 comprend plusieurs sources lumineuses 111a, 111b, 111c, trois dans cet exemple, à savoir une source centrale 111b, une première source latérale 111a placée d'un première côté de la source centrale 111b, et une deuxième source latérale 111c placée d'un deuxième côté de la source centrale 111b. Le plan A de la figure 5 correspond au plan orthogonal P des figures 2 à 4. Le plan A traverse la source centrale 111b.

Pour collimater la lumière émise par la source lumineuse 111 et émettre un éclairage homogène ou encore émettre un éclairage diffus, le système d'éclairage 110 pourra comprendre en outre un système optique de mise en forme 140 du faisceau lumineux émis par la source lumineuse 111, tel qu'une lentille ou une matrice de lentilles, des filtres, etc.

Pour ajuster la zone à éclairer, ce système optique de mise en forme 140 pourra également comprendre un masque, tel qu'une plaque avec un trou, pouvant être déplacé de sorte à laisser passer la lumière de la source lumineuse 111 pour que celle-ci éclaire la cellule vivante ou l'ensemble de cellules vivantes 80 à observer. Cela peut notamment est être le cas si la au moins une source lumineuse 111 est étendue ou si le système d'éclairage 110 comprend plusieurs sources lumineuses 111 pilotables séparément ou non. De plus l'utilisation d'un tel masque permet aussi de filtrer les lumières parasites qui pourraient perturber l'observation de la cellule vivante ou l'ensemble de cellules vivantes 80.

Ainsi, dans le cadre de la figure 5, pour réaliser un éclairage de type « texture » et éclairer la cellule vivante ou l'ensemble de cellules vivantes 80 à observer sur le côté, la source lumineuse 111b située à la verticale de l'embryon 80 pourrait être éteinte et l'une des deux sources lumineuses 111a ou 111c situées sur les côtés de la source lumineuse centrale 111b pourrait être allumée projetant ainsi une lumière oblique sur la cellule vivante ou l'ensemble de cellules vivantes 80.

Le système d'imagerie 120 est situé, dans le cas des figures 1 et 5, en dessous de la boîte de Petri 10 et donc de la cellule vivante ou l'ensemble de cellules vivantes 80 à observer (principe du microscope inversé). Il comprend la caméra grand champ 121 adaptée pour permettre d'identifier une cellule vivante ou un ensemble de cellules vivantes 80 à observer, la cellule vivante ou l'ensemble de cellules vivantes 80 étant déposés dans une boîte de Petri 10 adaptée.

Pour cela, la caméra grand champ 121 peut être réglée de telle sorte que celle-ci puisse imager un élément d'identification 50 présent sur la boîte de Petri 10 permettant ainsi l'identification de la cellule vivante ou l'ensemble de cellules vivantes 80 à observer. Pour cela, le système d'imagerie 120 peut comprendre en outre une unité de traitement qui analyse l'image capturée par la caméra grand champ 121 pour détecter la position de l'élément d'identification 50 et la position de la ou des cellules vivantes ou ensemble de cellules vivantes 80 à observer à l'aide des caractéristiques de la boîte de Petri 10.

Dans le cas où la cellule vivante ou l'ensemble de cellules vivantes 80 est un ou plusieurs embryons, cette unité de traitement permet aussi d'analyser l'image capturée par la caméra grand champ 121 lorsque celle-ci est positionnée, à l'aide des moyens de déplacement 160, de sorte à imager l'élément d'identification 50 de la boîte de Petri 10, de sorte à retrouver dans une base de données préalablement remplie les informations relatives au contenu de la boîte de Petri 10, c'est-à-dire à l'embryon ou aux embryons 80, telles que le couple de patients auquel appartiennent les embryons 80, le nombre d'embryons 80 présents dans la boîte, la date de préparation et de mise en place des embryons 80, etc.

Grâce à sa haute résolution, la caméra grand champ 121 est adaptée pour imager une cellule vivante ou ensemble de cellules vivantes 80 éclairé et pour permettre d'observer son développement. La caméra grand champ 121 est typiquement équipée d'un capteur d'image d'une résolution de l'ordre de 5 pixels par 1 µm observé et dotée de quantité de pixels permettant de couvrir toute la surface du puit observé, surface additionnée d'une surface assurant une marge de sécurité en prévision des incertitudes de positionnement.

De plus, de sorte à pouvoir séparer les différentes couches de cellules présentes dans la cellule vivante ou ensemble de cellules vivantes 80, la caméra grand champ 121 doit avoir une profondeur de champ relativement faible, de l'ordre de 100 µm.

Pour voir les différentes couches de cellules et donc observer la cellule vivante ou l'ensemble de cellules vivantes 80 dans sa profondeur, le dispositif d'imagerie 100 peut comprendre en outre une lentille liquide, adaptée pour contrôler la distance de focalisation de la cellule vivante ou l'ensemble de cellules vivantes 80 dans la direction de sa profondeur, c'est-à-dire selon un axe Z perpendiculaire au plan défini par la boîte de Petri 10 ou défini par le support 1. Par exemple, une lentille liquide 170 configurée par actionnement par électromouillage, ou utilisant une membrane à polymère électroactif, ou configurée par un actionneur piézoélectrique peut être utilisée. Dans le cas d'une lentille liquide 170 configurée par actionnement par électromouillage, une tension est utilisée pour modifier la forme de l'interface séparant deux liquides différentes, et modifie ainsi la focale de la lentille liquide 170. Dans le cas d'une lentille liquide 170 utilisant une membrane à polymère électroactif, la focale de la lentille liquide 170 est aussi modifiée par application d'une tension électrique entre une électrode et un substrat, modifiant ainsi la courbure de la lentille liquide 170. Dans le cas d'une lentille liquide 170 configurée par un actionneur piézoélectrique, celui-ci a pour effet de gonfler une membrane remplie de liquide. Le plan correspondant à la couche de cellules que l'on souhaite observé est ainsi configuré par configuration de la lentille liquide. Ainsi, l'avantage est de pouvoir s'affranchir d'un moyen de déplacement le long de l'axe z, dont l'actionnement est coûteux en temps.

Grâce à l'unité de traitement, le système d'imagerie 120 peut permettre de compter le nombre de cellules présentes dans la cellule vivante ou l'ensemble de cellules vivantes 80 observé. Pour cela, l'unité de traitement du système d'imagerie 120 peut analyser les images capturées par la caméra grand champ 121 et, par des traitements d'image tels que la détection de contours, permet de compter le nombre de cellules présentes dans la cellule vivante ou l'ensemble de cellules vivantes 80 observé.

Le comptage et notamment les traitements d'image sont optimum lorsque la cellule vivante ou l'ensemble de cellules vivantes 80 observé est éclairé à l'aide de l'éclairage de type « contours ».

Le système d'imagerie 120 peut permettre également de visualiser la texture et la granularité des cellules présentes dans l'embryon 80 observé. Pour cela, l'unité de traitement du système d'imagerie 120 analyse les images capturées par la caméra grand champ 121 et les traite avec des traitements d'image optimisant le rendu visuel de la texture et du grain des cellules présentes dans la cellule vivante ou l'ensemble de cellules vivantes 80 observé.

Comme illustré sur la figure 5, le système d'imagerie 120, et notamment la caméra grand champ 121, peuvent avantageusement être déportés à l'aide d'un système optique de renvoi 150, de sorte à rendre le dispositif 100 plus compact. Ce système optique de renvoi 150 peut être constitué par exemple d'une lentille 151 et d'un miroir de renvoi 152 positionné à 45° par rapport à l'axe optique A du dispositif d'imagerie 100, en pointillés sur la figure 5.

Dans le cas où la cellule vivante ou l'ensemble de cellules vivantes 80 est un embryon ou plusieurs embryons, comme l'illustre la figure 6, les dimensions du dispositif d'imagerie 100 sont de préférence compatibles avec les incubateurs 200 présents dans les centres de FIV pour que celui-ci puisse être disposé à l'intérieur de tels incubateurs 200. Ainsi, la hauteur et la profondeur du dispositif 100 peuvent être de l'ordre de 30 cm et la largeur du dispositif 100 peut être de l'ordre de 30 cm à 55 cm environ.

Le dispositif d'imagerie 100 de la figure 6 comporte un tiroir coulissant sur lequel peut être déposé une ou plusieurs boîtes de Petri 10 comme précédemment indiqué.

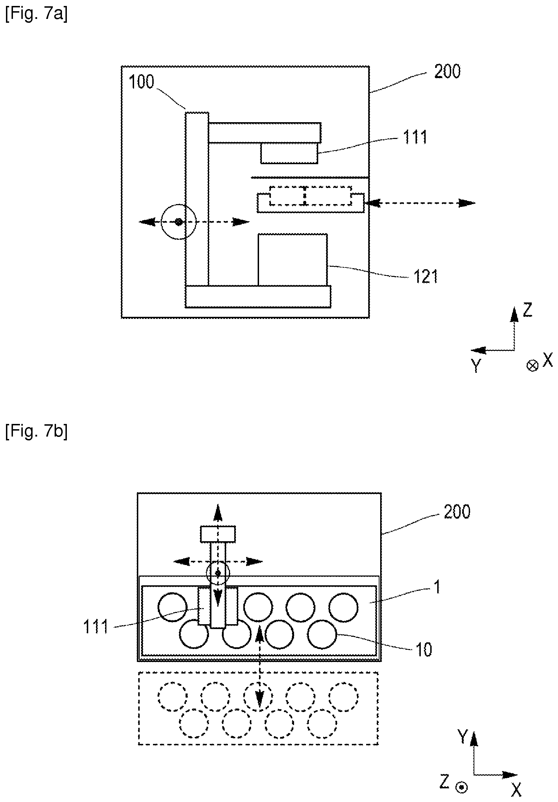

Les figures 7a et 7b représentent respectivement une vue de profil et une vue du dessus du dispositif d'imagerie 100 de la figure 6 présent dans un incubateur 200. Dans le cas présent, le support 1 faisant toute la largeur du dispositif d'imagerie 100, c'est l'ensemble formé par au moins le système d'éclairage 110 et la caméra grand champ 121 fixés sur une structure en forme de « U allongé » qui est déplacé par les moyens de déplacement relatif 160 dans un plan (X, Y) parallèle à celui défini par le support 1 de sorte à pouvoir visualiser soit un autre embryon 80 d'une même boîte de Petri 10, soit un embryon 80 dans une autre boîte de Petri 10.

### BOITE DE PETRI SELON L'INVENTION

Dans un deuxième temps une boîte de Petri 10 adaptée pour l'observation du développement de cellules vivantes ou ensembles de cellules vivantes 80 par un dispositif d'imagerie 100 tel que défini précédemment va être détaillée ci-dessous.

Selon un premier mode de réalisation, une telle boîte de Petri 10 peut comprendre :
- un récipient 20 adapté pour recevoir un ou plusieurs embryons 80 à observer ; et
- un couvercle 30.

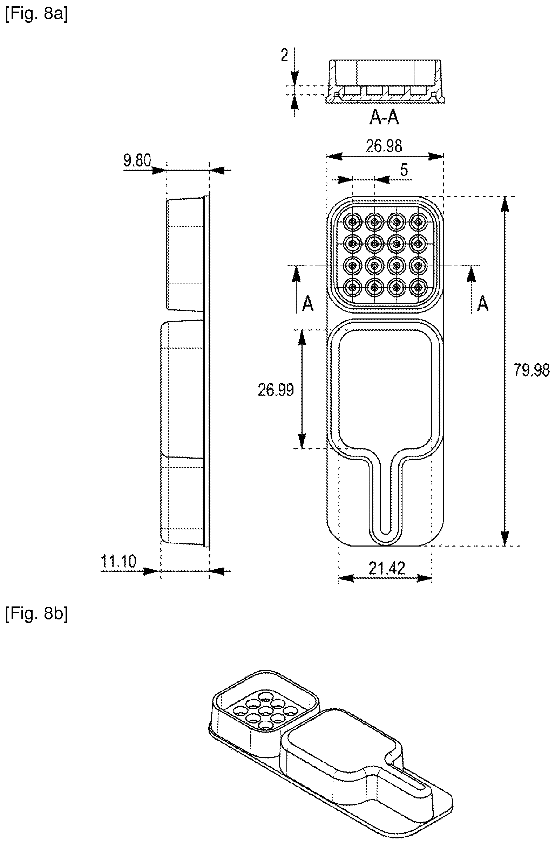

La figure 8a présente des vues bidimensionnelles de dessus et de profil ainsi qu'une coupe AA d'un exemple de boîte de Petri pouvant être utilisée dans le cadre de l'invention. On y observe un exemple de récipient 20 ainsi qu'un exemple de couvercle 30 correspondant. La figure 8b présente une représentation en relief du récipient 20 et du couvercle 30 de la figure 8a.

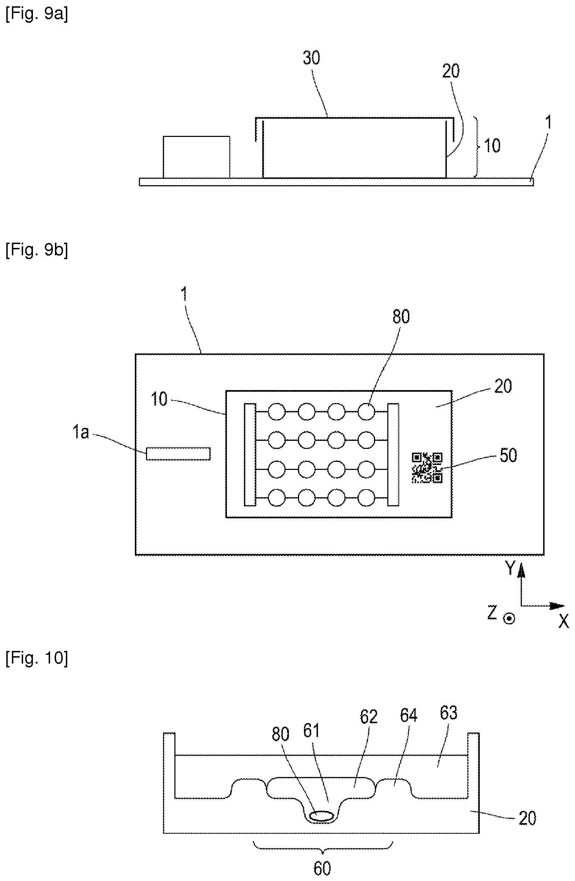

Une vue schématique de ce premier mode de réalisation de la boîte de Petri 10 est illustrée à la figure 9a.

De sorte à pouvoir voir les cellules vivantes ou ensembles de cellules vivantes 80 au travers de la boîte de Petri 10, celle-ci peut être fabriquée dans un matériau transparent à la lumière émise par le système d'éclairage 110 comme évoqué précédemment. Cette lumière est en général dans le domaine du visible (soit approximativement entre 400 nm et 800 nm). Ainsi le matériau utilisé peut être du verre ou un matériau plastique par exemple.

En référence à la figure 9b, le fond du récipient 20 comprend un élément d'identification 50 adapté pour permettre l'identification de la ou des cellules vivantes ou l'ensemble / les ensembles de cellules vivantes 80 observés.

Selon le mode de réalisation présenté dans la figure 9b, la boîte de Petri 10 est de forme rectangulaire.

L'élément d'identification 50 peut par exemple être un code-barres ou bien un code 2D, de type data-matrix comme représenté sur la figure 9b. Cet élément d'identification 50 permet notamment de connaître les informations relatives au contenu de la boîte de Petri 10, c'est-à-dire aux cellules vivantes ou ensembles de cellules vivantes 80 qu'elle contient. Dans le cas où la ou les cellules vivantes ou ensembles de cellules vivantes 80 sont un ou plusieurs embryons, les information relatives au contenu de la boîte de Petri 10 peuvent être par exemple le couple de patients auquel appartiennent les embryons 80, le nombre d'embryons 80 présents dans la boîte, la date de préparation et de mise en place des embryons 80, etc. au travers d'une base de données préalablement renseignée.

Selon ce mode de réalisation, les cellules vivantes ou ensembles de cellules vivantes 80 sont répartis de manière linéaire sur une ou plusieurs rangées comme c'est le cas pour la boîte de Petri 10 rectangulaire illustrée sur la figure 9b.

Selon un mode de réalisation particulier de la boîte de Petri 10 illustré à la figure 10, le fond du récipient 20 peut comprendre avantageusement au moins un réservoir à embryon 60 comprenant en son centre une cupule 61, ou encore logement, ou puits, adaptée pour recevoir une cellule vivante ou un ensemble de cellules vivantes 80, le réservoir 60 étant également adapté pour recevoir, en plus de la cellule vivante ou l'ensemble de cellules vivantes 80 dans la cupule 61, une goutte d'un milieu de culture 62 (typiquement de l'ordre de 6 µl). Le diamètre de la cupule peut être typiquement de l'ordre de 1 mm et sa capacité de l'ordre de 0,75 µl. La zone où se trouve le milieu de culture 62 est délimitée par une surélévation du fond du récipient 20 ou bosse 64.

L'ensemble formé par au moins la cellule vivante ou l'ensemble de cellules vivantes 80, la goutte de milieu de culture 62 peut être ensuite recouvert d'un liquide 63 tel que de l'huile ou de l'eau. Pour cela, les bords du récipient 20 doivent être suffisamment hauts, typiquement de l'ordre de 5 à 10 mm.

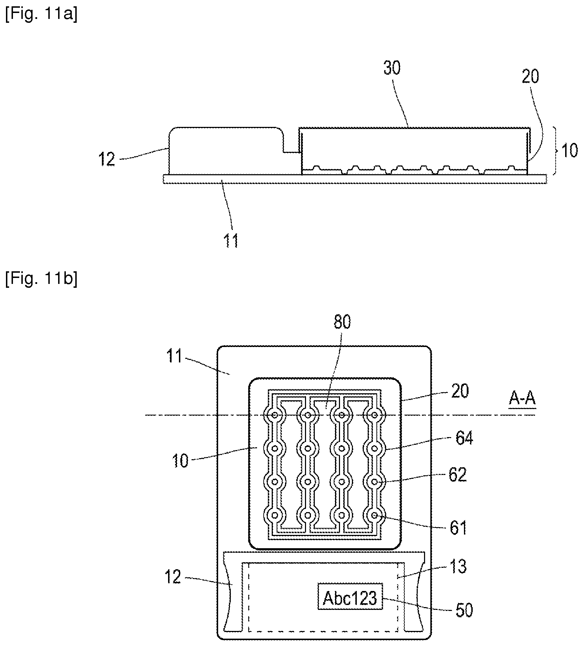

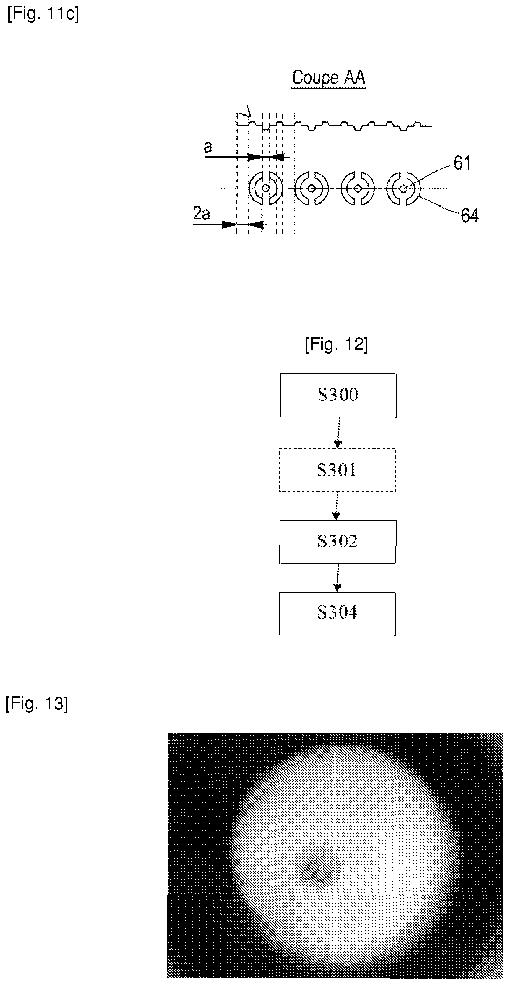

Selon un autre mode de réalisation, illustré dans les figures 11a à 11c et similaire à celui présenté dans les figures 9a et 9b, la boîte de Petri 10 peut être solidaire d'une plaque transparente 11 comprenant un ergo de préhension 12 permettant une manipulation aisée de la boîte Petri 10. Cela évite le risque de salir le fond du récipient 20 ou le couvercle 30 ce qui perturberait l'observation de l'embryon 80 et la qualité des images prises par le dispositif 100. Avantageusement, l'ergo de préhension 12 comprend une zone de marquage 13 sur laquelle il est possible d'apposer l'élément d'identification 50 plutôt que dans sur le fond du récipient 20.

Enfin, les bords du récipient 20 sont suffisamment hauts de sorte à pouvoir déposer une quantité de liquide 63, de type huile, ou de l'eau, pour recouvrir l'ensemble formé par la goutte de milieu de culture 62 et la cellule vivante ou l'ensemble de cellules vivantes 80, typiquement de l'ordre de 5 à 10 mm.

Selon le mode de réalisation, l'élément d'identification 50 peut être réalisé directement dans la matière, par usinage du fond du récipient 20 sur sa face interne ou externe, ou par apposition d'une étiquette et/ou de repères autocollants sur le fond extérieur du récipient 20.

### PROCEDE D'OBSERVATION SELON L'INVENTION

Dans un troisième temps un procédé d'observation du développement de cellules vivantes ou ensembles de cellules vivantes 80 par un dispositif d'imagerie 100 tel que défini précédemment va être détaillée ci-dessous.

Selon un premier mode de réalisation général du procédé illustré à la figure 12, celui-ci peut comprendre trois étapes principales :
- une étape de préparation S300 ;
- une étape d'identification S302 ; et
- une étape d'observation S304.

L'étape de préparation S300 consiste à déposer successivement une cellule vivante ou un ensemble de cellules vivantes 80 puis une goutte d'un milieu de culture 62 (ou inversement une goutte d'un milieu de culture 62 puis une cellule vivante ou un ensemble de cellules vivantes 80) puis à recommencer l'opération en fonction du nombre de cellules vivantes ou d'ensembles de cellules vivantes 80 à observer souhaité et enfin recouvrir le tout avec un liquide 63, de type huile, ou de l'eau, dans une boîte de Petri 10 telle que définie précédemment et pouvant être choisie parmi au moins une boîte de Petri disposée sur le support du dispositif.

La cellule vivante ou l'ensemble de cellules vivantes 80 peut être déposé dans la cupule 61 puis recouvert par une goutte d'un milieu de culture 62 dans le réservoir à cellule vivante ou ensemble de cellules vivantes 60.

Avantageusement, et notamment dans le cas où la cellule vivante ou ensemble de cellules vivantes 80 est un embryon ou plusieurs embryons, suite à cette opération de préparation ou de dépose des embryons 80, une base de données peut être remplie par le préparateur avec les caractéristiques de la boîte Petri 10 et surtout les données liées aux embryons 80 déposés dans la boîte de Petri 10 telles que : le couple de patients auquel appartiennent les embryons 80, le nombre d'embryons 80 présents dans la boîte, la date de préparation et de mise en place des embryons 80, etc.

L'étape d'identification S302 de la cellule vivante ou l'ensemble de cellules vivantes 80 à observer consiste à imager et analyser, à l'aide du système d'imagerie 120, l'élément d'identification 50 de la boîte de Petri 10 dans laquelle se trouve la cellule vivante ou ensemble de cellules vivantes 80 à observer.

Avantageusement, une étape optionnelle S301, dite étape de positionnement approximatif S301 peut être réalisée préalablement à l'étape, dite étape d'identification S302, ladite étape optionnelle S301consistant à déplacer relativement l'un par rapport à l'autre l'ensemble formé par le système d'éclairage 110 et la caméra grand champ 121 du système d'imagerie 120 et la boîte de Petri 10 à l'aide des moyens de déplacement 160, de sorte que la boîte de Petri 10 choisie soit approximativement alignée avec l'ensemble formé par le système d'éclairage (110) et la caméra grand champ (121) du système d'imagerie (120).

Ainsi, dans le cas où plusieurs boîtes de Petri 10 sont disposées sur le support, il suffit de positionner le système d'imagerie 120, lors d'une étape optionnelle S301, au - dessus d'une boîte de Petri choisie 10 pour que celle-ci soit directement identifiée, notamment par l'identification de l'élément d'identification 50 avec la caméra grand champ 121, en déplaçant l'ensemble formé par le système d'éclairage 110 et la caméra grand champ 121 système d'imagerie 120.

Puis l'unité de traitement du premier système d'imagerie 120 peut analyser l'image prise par la caméra grand champ 121 et permet d'analyser l'élément d'identification 50 pour remonter aux caractéristiques de la boîte de Petri 10 observée via une base de données préalablement remplie.

L'étape d'observation S304 consiste à imager une première cellule vivante ou ensemble de cellules vivantes 80 à l'aide du système d'imagerie 120.

Dans une configuration où le support 1 comprend des plots ou rebords, ou encore encoches permettant de pré-positionner une boîte de Petri 10 sur des positions prédéfinies, celle-ci y est ainsi pré-positionnée. Aucun réglage latéral préliminaire, c'est-à-dire, dans un plan parallèle au plan défini par le support 1, n'est utile pour positionner le système d'imagerie 120 et la caméra grand champ 121 relativement à la boîte de Petri 10 car le large champ de vue de la caméra grand champ 121 couvre au moins l'intégralité d'un logement 61, ou puits, de la boîte de Petri 10. Ceci permet, dès la mise en fonctionnement du système d'imagerie 120, après avoir défini le plan d'observation de la cellule vivante ou ensemble de cellules vivantes 80 par configuration de la caméra grand champ 121, de repérer directement une cellule vivante ou un ensemble de cellules vivantes 80 positionnée dans l'un des logements 61 de la boîte de Petri 10 en place, quelle que soit la position de la cellule vivante ou ensemble de cellules vivantes 80 dans son logement 61. Ainsi, le système d'éclairage 110 éclaire la première cellule vivante ou ensemble de cellules vivantes 80 et la caméra grand champ 121 image celui-ci. La figure 13 montre une image plein d'un embryon obtenue par le procédé d'observation précédemment décrit, l'image étant notamment obtenue après l'étape S304. On peut y observer l'embryon positionné du côté gauche de son logement 61 dans la boîte de Petri 10 et distinguer les cellules qui le constituent.

Avantageusement, lors de cette étape d'observation S304, il sera possible de compter le nombre de cellules présentes dans la cellule vivante ou ensemble de cellules vivantes 80 observé ou d'observer la texture et le grain de ces cellules vivantes ou ensembles de cellules vivantes grâce au système d'imagerie 120 et éventuellement aussi grâce à un éclairage adapté.

Dans le cas particulier où la cellule vivante ou ensemble de cellules vivantes 80 est un embryon, pour pouvoir en observer l'évolution du développement dans son ensemble les images prises par la caméra grand champ 131 pourront être enregistrées en prenant soin d'identifier l'embryon 80 et la date et l'heure de la prise de vue.

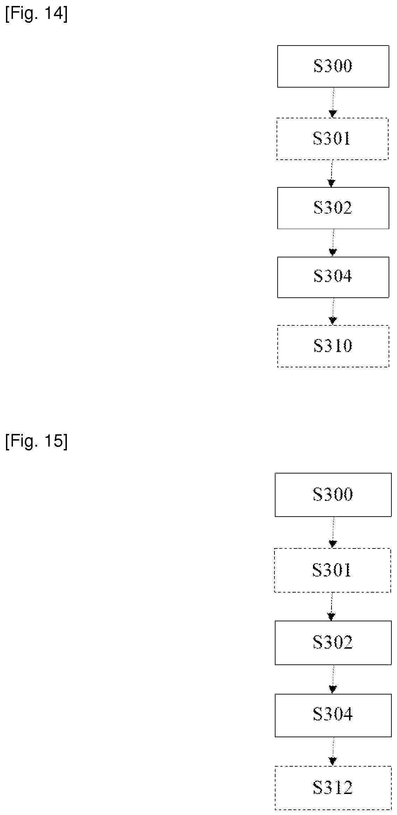

Selon un deuxième mode de réalisation général du procédé d'observation selon l'invention, et illustré à la figure 14, il est possible d'observer une autre cellule vivante ou ensemble de cellules vivantes 80 située dans un autre logement 61 de la boîte de Petri10. Une étape supplémentaire S310 peut ainsi être effectuée par rapport au premier mode de réalisation du procédé d'observation dont un synoptique a été illustré à la figure 12. La boîte de Petri 10 et de l'ensemble formé par au moins le système d'éclairage 110 et la caméra petit champ 131 peuvent être déplacés relativement l'un par rapport à l'autre avec les moyens de déplacement 160. Le déplacement est réalisé selon un plan parallèle au plan horizontal défini par la boîte de Petri 10 ou à celui défini par le support 1 sur lequel est posée la boîte de Petri 10. Le déplacement est effectué de sorte à ce que l'ensemble formé par au moins le système d'éclairage 110 et la caméra grand champ 121 soit positionné au niveau du logement 61 de la nouvelle cellule vivante ou ensemble de cellules vivantes 80 à observer. La figure 14 présente le synoptique de ce mode de réalisation d'observation d'une autre cellule vivante ou ensemble de cellules vivantes 80 située dans un autre logement 61 de la boîte de Petri.

Il est à noter que pour gagner du temps lors de l'observation de plusieurs cellules vivantes ou ensembles de cellules vivantes 80, l'unité de traitement du système d'imagerie 120 peut conserver en mémoire les positions précédentes des cellules vivantes ou ensembles de cellules vivantes 80 observés pour pouvoir commander aux moyens de déplacement d'aller directement à ces positions. Ainsi, le time-lapse entre deux prises d'image d'une même cellule vivante ou ensemble de cellules vivantes 80 est diminué et l'évolution temporelle de la cellule vivante ou ensemble de cellules vivantes 80 est observée plus efficacement.

Dans le cas où le dispositif d'imagerie 100 comprend en outre une lentille liquide 170, le plan d'observation de la cellule vivante ou ensemble de cellules vivantes 80 peut être déterminé par une configuration de la lentille liquide 170.

Selon un troisième mode de réalisation du procédé d'observation selon l'invention, on peut observer une cellule vivante ou un ensemble de cellules vivantes 80 dans sa profondeur. On utilise pour ce faire un dispositif d'imagerie 100 comprenant une lentille liquide 170. L'utilisation de celle-ci permet de s'affranchir d'un moyen de déplacement mécanique le long de l'axe z défini perpendiculairement au plan horizontal défini par le support 1, et donc de réduire le temps de configuration du dispositif d'imagerie pour l'observation de cellules vivantes ou ensembles de cellules vivantes 80. Typiquement la plage de profondeur d'une cellule vivante ou ensemble de cellules vivantes 80 explorée est de l'ordre de quelques dizaines de micromètres (par exemple de 10 µm à 100 µm, typiquement de 20 µm). Un plan d'observation est déterminé par une configuration de la lentille liquide 170 dans une étape S312. Par configuration de la lentille liquide 170, il doit être compris le réglage d'un paramètre de contrôle tel qu'une tension électrique permettant de définir la focale de la lentille liquide 170. Par exemple, pour mettre en œuvre le mode de réalisation du procédé d'observation précédemment décrit, le modèle de lentille liquide C-u-25H0-075 de la marque Varioptic, peut être utilisé.

Ainsi, l'étape supplémentaire S312 de détermination d'un plan d'observation par configuration de la lentille liquide 170 peut être répétée plusieurs fois pour observer une cellule vivante ou ensemble de cellules vivantes 80 en plusieurs plans perpendiculaires à la direction de la profondeur déterminés par différentes configurations de la lentille liquide 170. La figure 15 présente le synoptique de ce mode de réalisation d'observation d'une cellule vivante ou ensemble de cellules vivantes 80 dans sa profondeur.

### PRODUIT PROGRAMME D'ORDINATEUR SELON L'INVENTION

L'invention porte également un produit programme d'ordinateur téléchargeable depuis un réseau de communication et/ou enregistré sur un support lisible par ordinateur et/ou exécutable par un processeur. Ce programme d'ordinateur comprend des instructions de code de programme pour la mise en œuvre du procédé d'observation du **développement** d'une cellule vivante ou d'un ensemble de cellules vivantes 80 tel que précédemment défini.

Pour optimiser l'observation des cellules vivantes ou d'ensembles de cellules vivantes 80 et notamment réduire au maximum l'écart entre chaque prise de vue par la caméra grand champ 121, le programme ordinateur pourra optimiser le déplacement de l'ensemble formé par au moins le système d'éclairage 110 et la caméra grand champ 121 grâce à la connaissance de positions précédemment enregistrées des cellules vivantes ou d'ensembles de cellules vivantes 80.

Avantageusement, ce programme d'ordinateur pourra également permettre de visionner les images prises d'une cellule vivante ou d'un ensemble de cellules vivantes 80 soit sous forme d'une vidéo retraçant son évolution au fil du temps, soit image par image.

### EXEMPLES

EXEMPLE 1 : Observation d'un embryon avec un dispositif d'imagerie selon l'invention et par un procédé d'observation selon l'invention avec différents types d'éclairages

La figure 16 montre deux images d'un embryon 80 obtenues avec un dispositif d'imagerie 100 selon l'invention et selon le premier mode de réalisation du procédé d'observation selon l'invention, en utilisant respectivement un éclairage de type « contours » et un éclairage de type « texture ». On y distingue respectivement, dans l'image en éclairage de type « contours », les bords des cellules constituant l'embryon 80 ainsi que les bords de celui-ci, et dans l'image en éclairage de type « texture », le relief et la texture de ces éléments.

EXEMPLE 2 : Observation d'un embryon dans sa profondeur avec un dispositif d'imagerie selon l'invention et selon le troisième mode de réalisation du procédé d'observation selon l'invention

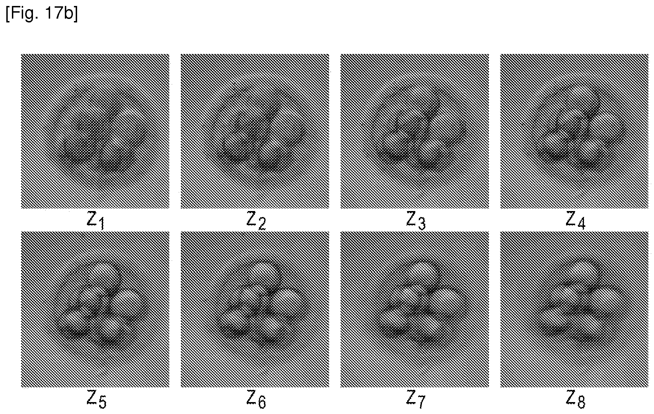

Les figures 18a et 18b montrent différentes coupes transversales d'un embryon à différentes profondeurs, obtenues avec un dispositif d'imagerie comprenant un modèle de lentille liquide de la marque Varioptic, (C-u-25H0-07), présentant une distance focale de 7,5mm pour une tension d'application de 40V. La figure 17a présente une série de coupes avec un éclairage de type « contours », où les plans de coupe sont espacés d'environ 30 microns par application de plages de tensions déterminées de la lentille liquide. La figure 17b présente une série de coupes avec un éclairage de type relief, où les plans de coupe sont espacés de 30µm. On peut observer dans ces séries d'images les mêmes propriétés que dans la figure 16, et ainsi que la netteté de chaque coupe transversale.

L'invention telle que précédemment décrite présente de multiples avantages.

Le dispositif d'imagerie n'est plus équipé d'un jeu d'objectifs de grossissements différents à manipuler manuellement comme sur les microscopes actuels mais il est équipé d'une unique caméra permettant l'observation directe, sans réglage préliminaire ni déplacements d'une première cellule vivante ou ensemble de cellules vivantes 80 placés dans une position quelconque dans un logement 61 d'une boîte de Petri 10, l'observation étant une observation en haute résolution de la cellule ou l'ensemble de cellules vivantes 80.

Les contraintes vis-à-vis de la dépose des cellules vivantes ou ensembles de cellules vivantes 80 qui peuvent se déplacer au sein de leur logement 61 dans la boîte de Petri 10 sont donc relâchées par le fait que la caméra grand champ 121 couvre au moins la totalité de la surface d'un logement 61. Ceci permet de ne pas avoir à déplacer le dispositif d'imagerie 100 pour observer la ou les cellules vivantes ou ensembles de cellules vivantes 80 dans leur logement 61.

Les différents types d'éclairage que peut comprendre le dispositif d'imagerie permettent de configurer en conséquence les traitements d'image associés aux différentes phases du processus d'observation telles que l'identification de la boîte de Petri 10 et donc des cellules vivantes ou ensembles de cellules vivantes 80, leur détection par le système d'imagerie 120, ou encore le comptage du nombre de cellules présentes dans la cellule vivante ou l'ensemble de cellules vivantes 80 observé.

Le dispositif d'imagerie 100 et le procédé d'observation de cellules vivantes ou d'ensembles de cellules vivantes présentés ici permettent d'améliorer la qualité optique et d'image, ainsi que l'efficacité matérielle, c'est-à-dire l'utilisation d'un matériel simplifié par rapport aux dispositifs connus dans l'art antérieur, et temporelle, grâce à la limitation des moyens de déplacement et à la suppression des déplacements en profondeur, des processus d'étude de la reproduction, notamment de la Fécondation *In Vitro,* tout en réduisant les coûts de fabrication et d'utilisation du dispositif.]

## Revendications

1. Dispositif d'imagerie (100) pour l'observation du développement de cellules vivantes ou d'ensembles de cellules vivantes (80) dans le cadre de l'étude de la reproduction et en particulier pour la Fécondation In Vitro (FIV), **caractérisé en ce qu'**il comprend :
- un système d'imagerie (120) comprenant une caméra grand champ (121) adaptée à l'observation d'une ou plusieurs cellules vivantes ou ensembles de cellules vivantes (80) à observer, les cellules vivantes ou ensembles de cellules vivantes (80) étant déposés dans une boîte de Petri (10) contenant un logement spécifique (61) pour chaque cellule vivante ou ensemble de cellules vivantes (80) ;
- un support (1) apte à recevoir la boîte de Petri (10) et positionné entre le système d'éclairage (110) et le système d'imagerie (120) ;
- un système d'éclairage (110) comprenant trois sources lumineuses (111a, 111b, 111c), adaptées pour éclairer un objet qui est une cellule vivante ou un ensemble de cellules vivantes (80) à observer, dont une source centrale (111b), une première source latérale (111a) placée d'un première côté de la source centrale (111b), et une deuxième source latérale (111c) placée d'un deuxième côté de la source centrale (111b),
le système d'éclairage (110) étant configuré pour mettre en œuvre chacun des types d'éclairage particuliers suivants :
- un éclairage de type « repérage » permettant de repérer la ou les cellules vivantes ou ensembles de cellules vivantes (80) à observer par la caméra grand champ (121) du système d'imagerie (120),
ledit éclairage de type « repérage » étant réalisé en mettant en œuvre la source centrale (111b), la première source latérale (111a) et la deuxième source latérale (111c), les rayons lumineux (112) issus des sources lumineuses (111a, 111b, 111c) étant focalisés sur l'objet selon un cône (α_GC ) ayant une ouverture angulaire comprise entre 26° et 34° ;
- un éclairage de type « contours » permettant de compter la ou les cellules présentes dans la cellule vivante ou l'ensemble de cellules vivantes (80) observé par la caméra grand champ (121) du système d'imagerie (120), ledit éclairage de type « contours » étant réalisé en mettant en œuvre la première source latérale (111a) et la deuxième source latérale (111c), les rayons issus de la première source latérale (111a) et de la deuxième source latérale (111c) étant séparés en deux faisceaux collimatés (113, 114) symétriques par rapport à l'axe perpendiculaire (P) au plan formé par le support (1) de la boîte de Petri (10) ou la boîte de Petri elle-même, un premier faisceau (113) issu de la première source latérale (111a) éclairant l'objet selon un angle d'incidence par rapport à l'axe perpendiculaire (P) comprise entre 10° et 14°, un deuxième faisceau (114) issu de la deuxième source latérale (111c) éclairant l'objet selon un angle d'incidence par rapport à l'axe perpendiculaire (P) comprise entre 10° et 14°;
- un éclairage de type « relief » permettant de visualiser la texture et la granularité de la ou des cellules présentes dans la cellule vivante ou l'ensemble de cellules vivantes (80) observé par la caméra grand champ (121) du système d'imagerie (120), ledit éclairage de type « relief » étant réalisé en mettant en œuvre la première source latérale (111a), un unique faisceau lumineux (115) collimaté issu de la première source latérale (111a) se propageant selon un axe incliné d'un angle par rapport à l'axe perpendiculaire (P) compris entre 8° et 16°;
- des moyens de déplacement relatif (160) de la boîte de Petri (10) par rapport à l'ensemble formé par le système d'éclairage (110) et ladite caméra grand champ (121) de sorte à pouvoir observer des cellules vivantes ou ensembles de cellules vivantes (80) situés dans les différents logements de la boîte de Petri (10),
ladite caméra grand champ (121) présentant un champ de vue couvrant au moins la surface totale d'un des logements de la boîte de Petri (10), et ledit dispositif d'imagerie (100) étant adapté pour imager, sans déplacement relatif de ladite boîte de Petri avec les moyens de déplacement (160) par rapport à l'ensemble formé par le système d'éclairage (110) et ladite caméra grand champ (121), une cellule vivante ou un ensemble de cellules vivantes dans une position quelconque dans son logement dans la boîte de Petri (10) et ladite caméra grand champ (121) présentant une résolution adaptée à l'observation des détails d'une cellule vivante ou d'un ensemble de cellules vivantes (80), lesdits détails ayant une taille micrométrique.

2. Dispositif d'imagerie (100) selon la revendication 1 dans lequel le ou les ensembles de cellules vivantes sont un ou plusieurs embryons.

3. Dispositif d'imagerie (100) selon l'une quelconque des revendications 1 à 2, dans lequel les moyens de déplacement (160) sont aptes à déplacer l'ensemble formé par le système d'éclairage (110) et la caméra grand champ (121) du système d'imagerie (120) relativement par rapport à la boîte de Petri (10) dans laquelle sont déposés les cellules vivantes ou ensembles de cellules vivantes (80) à observer.

4. Dispositif d'imagerie (100) selon l'une quelconque des revendications 1 à 3 comprenant en outre une lentille liquide (170), adaptée pour contrôler une distance de focalisation dans la direction de la profondeur de la cellule vivante ou de l'ensemble de cellules vivantes.

5. Procédé d'observation du développement de cellules vivantes ou ensembles de cellules vivantes par un dispositif d'imagerie (100) tel que défini dans l'une des revendications 1 à 4 comprenant les étapes suivantes :
- une étape de préparation (S300) consistant à déposer successivement une cellule vivante ou un ensemble de cellules vivantes dans une boîte de Petri (10) comprenant :
- un récipient (20) adapté pour recevoir une ou plusieurs cellules vivantes ou ensembles de cellules vivantes à observer,
- un couvercle (30),
- un élément d'identification (50) adapté à l'identification de la ou des cellules vivantes ou ensembles de cellules vivantes observés,
- des logements ou puits spécifiques destinés à la dépose d'une cellule vivante ou d'un ensemble de cellules vivantes, lesdits logements ou puits étant réalisés sous forme de cupules (61) adaptées pour recevoir, en plus de la cellule vivante ou l'ensemble de cellules vivantes dans la cupule, une goutte d'un milieu de culture (62).,
puis une goutte d'un milieu de culture (62) dans ladite boîte de Petri (10), l'opération étant réitérée autant de fois que nécessaire en fonction du nombre de cellules vivantes ou ensembles de cellules vivantes à observer souhaité ; puis à recouvrir le tout avec un liquide (63), tel que de l'huile ou de l'eau;
- une étape d'identification (S302) de la boîte de Petri (10) consistant à détecter et visualiser, à l'aide d'un lecteur dédié, l'élément d'identification (50) de la boîte de Petri (10) dans laquelle se trouve la ou les cellules vivantes ou ensembles de cellules vivantes (80) à observer ; et
- une étape d'observation (S304) d'une première cellule vivante ou ensemble de cellules vivantes (80) à l'aide du système d'imagerie (120).

6. Procédé d'observation du développement de cellules vivantes ou ensembles de cellules vivantes selon la revendication 5 comprenant en outre, préalablement à l'étape d'identification (S302), une étape de positionnement approximatif (S301) de la boîte de Petri (10) choisie par rapport à l'ensemble formé par le système d'éclairage (110) et la caméra grand champ (121) du système d'imagerie (120) relativement à une boîte de Petri 10 choisie à l'aide des moyens de déplacement, de sorte que la boîte de Petri (10) soit approximativement alignée avec l'ensemble formé par le système d'éclairage (110) et la caméra grand champ (121) du système d'imagerie (120).

7. Procédé d'observation du développement de cellules vivantes ou ensembles de cellules vivantes selon la revendication 5 ou 6, comprenant, après l'étape d'observation d'une première cellule vivante ou d'un premier ensemble de cellules vivantes, une étape de déplacement relatif (S310) de la boîte de Petri (10) par rapport à l'ensemble formé par le système d'éclairage (110) et la caméra grand champ (121) du système d'imagerie (120) permettant l'observation d'une autre cellule vivante ou d'un autre ensemble de cellules vivantes situé dans un autre logement (61) de la boîte de Petri (10).

8. Procédé d'observation du développement de cellules vivantes ou ensembles de cellules vivantes selon l'une quelconque des revendications 5 à 7, dans le cas où le dispositif d'imagerie (100) comprend en outre une lentille liquide (170), dans lequel l'étape d'observation d'une première cellule vivante ou d'un premier ensemble de cellules vivantes (80) est effectuée dans un plan d'observation horizontal parallèle au support (1) apte à recevoir la boîte de Petri (10) et perpendiculaire à la direction de la profondeur de ladite première cellule vivante ou dudit ensemble de cellules vivantes , ledit plan étant déterminé dans une étape supplémentaire de détermination (S312) par une configuration de la lentille liquide.

9. Procédé d'observation du développement de cellules vivantes ou ensembles de cellules vivantes selon la revendication 8, dans lequel l'étape de détermination d'un plan d'observation (S312) de ladite première cellule vivante ou dudit ensemble de cellules vivantes (80) est répétée pour différents plans perpendiculaires à la direction de la profondeur de ladite première cellule vivante ou dudit ensemble de cellules vivantes (80) par différentes configurations de la lentille liquide, de sorte à observer une première cellule vivante ou un premier ensemble de cellules vivantes (80) dans les différents plans d'observation déterminés.

## Patentansprüche

1. Bildgebungsvorrichtung (100) zur Beobachtung der Entwicklung von lebenden Zellen oder Sätzen lebender Zellen (80) im Rahmen der Untersuchung der Fortpflanzung und insbesondere für die In-vitro-Fertilisation (IVF), **dadurch gekennzeichnet, dass** sie umfasst:
- ein Bildgebungssystem (120), umfassend eine Weitwinkelkamera (121), die zur Beobachtung von einer oder mehreren zu beobachtenden lebenden Zellen oder Sätzen lebender Zellen (80) ausgelegt ist, wobei die lebenden Zellen oder Sätze lebender Zellen (80) in einer Petrischale (10) angeordnet sind, die für jede lebende Zelle oder jeden Satz lebender Zellen (80) eine spezifische Aussparung (61) enthält;
- eine Halterung (1), die zur Aufnahme der Petrischale (10) geeignet ist und zwischen dem Beleuchtungssystem (110) und dem Bildgebungssystem (120) angeordnet ist;
- ein Beleuchtungssystem (110) umfassend drei Lichtquellen (111a, 111b, 111c), die ausgelegt sind, um ein zu beobachtendes Objekt, welches eine lebende Zelle oder ein Satz lebender Zellen (80) ist, zu beleuchten, darunter eine zentrale Quelle (111b), eine erste seitliche Quelle (111a), die auf einer ersten Seite der zentralen Quelle (111b) platziert ist, und eine zweite seitliche Quelle (111c), die auf einer zweiten Seite der zentralen Quelle (111b) platziert ist,
wobei das Beleuchtungssystem (110) konfiguriert ist, um jede der folgenden spezifischen Beleuchtungsarten umzusetzen:
- eine Beleuchtung vom Typ "Lokalisierung" zum Lokalisieren der von der Weitwinkelkamera (121) des Bildgebungssystems (120) zu beobachtende(n) lebenden Zelle(n) oder der Sätze lebender Zellen (80), wobei die Beleuchtung vom Typ "Lokalisierung" durch Einsatz der zentralen Quelle (111b), der ersten seitlichen Quelle (111a) und der zweiten seitlichen Quelle (111c) erreicht wird, wobei die von den Lichtquellen (111a, 111b, 111c) ausgehenden Lichtstrahlen (112) auf das Objekt in einem Kegel (α_GC) mit einer Winkelöffnung zwischen 26° und 34° fokussiert werden;
- eine Beleuchtung vom Typ "Kontur" zur Zählung der Zelle(n), die in der lebenden Zelle oder dem Satz lebender Zellen (80) vorhanden sind, die von der Weitwinkelkamera (121) des Bildgebungssystems (120) beobachtet werden, wobei die Beleuchtung vom Typ "Kontur" durch den Einsatz der ersten seitlichen Quelle (111a) und der zweiten seitlichen Quelle (111c) erreicht wird, wobei die von der ersten seitlichen Quelle (111a) und der zweiten seitlichen Quelle (111c) ausgehenden Strahlen in zwei kollimierte Strahlenbündel (113, 114) aufgeteilt werden, die symmetrisch zur Achse (P) sind, die senkrecht zu der Ebene steht, die durch die Halterung (1) der Petrischale (10) oder die Petrischale selbst gebildet wird, wobei ein erstes Strahlenbündel (113) von der ersten seitlichen Quelle (111a) das Objekt unter einem Einfallswinkel zur senkrechten Achse (P) zwischen 10° und 14° beleuchtet, wobei ein zweites Strahlenbündel (114), das von der zweiten seitlichen Quelle (111c) ausgeht, das Objekt unter einem Einfallswinkel zur senkrechten Achse (P) zwischen 10° und 14° beleuchtet;
- eine Beleuchtung vom Typ "Relief" zur Visualisierung der Textur und Körnigkeit der Zelle(n), die in der lebenden Zelle oder dem Satz lebender Zellen (80) vorhanden sind, die von der Weitwinkelkamera (121) des Bildgebungssystems (120) beobachtet werden, wobei die Beleuchtung vom Typ "Relief" durch den Einsatz der ersten seitlichen Quelle (111a) erreicht wird, wobei sich ein einziger kollimierter Lichtstrahl (115), ausgehend von der ersten seitlichen Quelle (111a), entlang einer Achse ausbreitet, die gegenüber der senkrechten Achse (P) um einen Winkel zwischen 8° und 16° geneigt ist;
- Mittel (160) zur relativen Verschiebung der Petrischale (10) gegenüber der aus dem Beleuchtungssystem (110) und der Weitwinkelkamera (121) gebildeten Anordnung, um lebende Zellen oder Sätze lebender Zellen (80) in den verschiedenen Aussparungen der Petrischale (10) beobachten zu können,
wobei die Weitwinkelkamera (121) ein Sichtfeld aufweist, das mindestens die gesamte Oberfläche einer der Aussparungen der Petrischale (10) abdeckt, und wobei die Bildgebungsvorrichtung (100) ausgelegt ist, um ohne relative Verschiebung der Petrischale mit den Verschiebungsmitteln (160) gegenüber der aus dem Beleuchtungssystem (110) und der Weitwinkelkamera (121) gebildeten Anordnung eine lebende Zelle oder einen Satz lebender Zellen an einer beliebigen Position in ihrer Aussparung in der Petrischale (10) abzubilden, und wobei die Weitwinkelkamera (121) eine Auflösung aufweist, die zur Beobachtung von Details einer lebenden Zelle oder eines Satzes lebender Zellen (80) ausgelegt ist, wobei die Details eine mikrometrische Größe haben.

2. Bildgebungsvorrichtung (100) nach Anspruch 1, wobei der oder die Sätze lebender Zellen ein oder mehrere Embryonen sind.

3. Bildgebungsvorrichtung (100) nach einem der Ansprüche 1 bis 2, wobei die Verschiebungsmittel (160) geeignet sind, um die aus dem Beleuchtungssystem (110) und der Weitwinkelkamera (121) des Bildgebungssystems (120) gebildete Anordnung relativ zu der Petrischale (10) zu verschieben, in der die zu beobachtenden lebenden Zellen oder Sätze lebender Zellen (80) angeordnet sind.

4. Bildgebungsvorrichtung (100) nach einem der Ansprüche 1bis 3, ferner umfassend eine Flüssigkeitslinse (170), die ausgelegt ist, um einen Brennweitenabstand in der Tiefenrichtung der lebenden Zelle oder dem Satz lebender Zellen zu steuern.

5. Verfahren zur Beobachtung der Entwicklung von lebenden Zellen oder Sätzen lebender Zellen durch eine Bildgebungsvorrichtung (100) wie in einem der Ansprüche 1 bis 4 definiert, umfassend die folgenden Schritte:
- einen Vorbereitungsschritt (S300), bestehend aus dem nacheinander erfolgenden Ablegen einer lebenden Zelle oder eines Satzes lebender Zellen in eine Petrischale (10), umfassend:
- einen Behälter (20), der ausgelegt ist, um eine oder mehrere zu beobachtende lebende Zellen oder Sätze lebender Zellen aufzunehmen,
- einen Deckel (30),
- ein Identifizierungselement (50), das zur Identifizierung der beobachteten lebenden Zelle(n) oder Sätze lebender Zellen ausgelegt ist,
- spezifische Aussparungen oder Vertiefungen zur Ablage einer lebenden Zelle oder eines Satzes lebender Zellen, wobei die Aussparungen oder Vertiefungen in Form von Mulden (61) ausgebildet sind, die ausgelegt sind, um zusätzlich zu der lebenden Zelle oder dem Satz lebender Zellen in der Mulde einen Tropfen eines Nährmediums (62) aufzunehmen,
dann einen Tropfen eines Nährmediums (62) in der Petrischale (10), wobei dieser Vorgang so oft wie nötig wiederholt wird, je nach der gewünschten Anzahl der zu beobachtenden lebenden Zellen oder Sätzen von Zellen; dann zum Bedecken des Ganzen mit einer Flüssigkeit (63), wie beispielsweise Öl oder Wasser;
- einen Identifizierungsschritt (S302) der Petrischale (10), bestehend darin, das Identifikationselement (50) der Petrischale (10), in der sich die zu beobachtende(n) lebende(n) Zelle(n) oder Sätze lebender Zellen (80) befindet/befinden, mittels eines speziellen Lesegeräts zu erfassen und zu visualisieren; und
- einen Beobachtungsschritt (S304) einer ersten lebenden Zelle oder eines Satzes lebender Zellen (80) mittels des Bildgebungssystems (120).

6. Verfahren zur Beobachtung der Entwicklung von lebenden Zellen oder Sätzen lebender Zellen nach Anspruch 5, ferner umfassend, vor dem Identifizierungsschritt (S302), einen Schritt der ungefähren Positionierung (S301) der ausgewählten Petrischale (10) gegenüber der Anordnung, die aus dem Beleuchtungssystem (110) und der Weitwinkelkamera (121) des Bildgebungssystems (120) relativ zu einer Petrischale (10) gebildet wird, die mittels der Verschiebungsmittel ausgewählt wird, sodass die Petrischale (10) annähernd auf die aus dem Beleuchtungssystem (110) und der Weitwinkelkamera (121) des Bildgebungssystems (120) gebildete Anordnung ausgerichtet ist.

7. Verfahren zur Beobachtung der Entwicklung von lebenden Zellen oder Sätzen lebender Zellen nach Anspruch 5 oder 6, umfassend nach dem Schritt der Beobachtung einer ersten lebenden Zelle oder eines ersten Satzes lebender Zellen einen Schritt der relativen Verschiebung (S310) der Petrischale (10) gegenüber der aus dem Beleuchtungssystem (110) und der Weitwinkelkamera (121) des Bildgebungssystems (120) gebildeten Anordnung, wodurch die Beobachtung einer weiteren lebenden Zelle oder eines weiteren Satzes lebender Zellen ermöglicht wird, die sich in einer anderen Aussparung (61) der Petrischale (10) befindet.

8. Verfahren zur Beobachtung der Entwicklung von lebenden Zellen oder von Sätzen lebender Zellen nach einem der Ansprüche 5 bis 7, wobei, wenn die Bildgebungsvorrichtung (100) ferner eine Flüssiglinse (170) umfasst, der Schritt der Beobachtung einer ersten lebenden Zelle oder eines ersten Satzes lebender Zellen (80) in einer horizontalen Beobachtungsebene durchgeführt wird, die parallel zu der Halterung (1) verläuft, die zur Aufnahme der Petrischale (10) geeignet ist, und senkrecht zur Tiefenrichtung der ersten lebenden Zelle oder des Satzes lebender Zellen verläuft, wobei die Ebene in einem zusätzlichen Bestimmungsschritt (S312) durch eine Konfiguration der Flüssiglinse bestimmt wird.

9. Verfahren zur Beobachtung der Entwicklung von lebenden Zellen oder von Sätzen lebender Zellen nach Anspruch 8, wobei der Schritt der Bestimmung einer Beobachtungsebene (S312) der ersten lebenden Zelle oder des Satzes lebender Zellen (80) für verschiedene Ebenen senkrecht zur Tiefenrichtung der ersten lebenden Zelle oder des Satzes lebender Zellen (80) durch verschiedene Konfigurationen der Flüssiglinsen wiederholt wird, um eine erste lebende Zelle oder einen ersten Satz lebender Zellen (80) in den verschiedenen bestimmten Beobachtungsebenen zu beobachten.

## Claims

1. Imaging device (100) for observing the development of living cells or sets of living cells (80) in the context of studying reproduction and in particular for In Vitro Fertilisation (IVF), **characterised in that** it comprises:
- an imaging system (120) comprising a wide-field camera (121) adapted for observing one or more living cells or sets of living cells (80) to be observed, the living cells or sets of living cells (80) being deposited in a Petri dish (10) containing a specific compartment (61) for each living cell or set of living cells (80);
- a support (1) for receiving the Petri dish (10), which is positioned between the lighting system (110) and the imaging system (120);
- a lighting system (110) comprising three light sources (111a, 111b, 111c), adapted for illuminating an object which is a living cell or a set of living cells (80) to be observed, including a central source (111b), a first lateral source (111a) placed on a first side of the central source (111b), and a second lateral source (111c) placed on a second side of the central source (111b),
the lighting system (110) being configured to implement each of the following specific types of lighting:
- a "locating" type of lighting for locating the living cell or cells or sets of living cells (80) to be observed by the wide-field camera (121) of the imaging system (120), said "locating" type of lighting being produced using the central source (111b), the first lateral source (111a) and the second lateral source (111c), the light rays (112) from the light sources (111a, 111b, 111c) being focused on the object along a cone (α_GC) having an angular aperture of between 26° and 34°;
- a "contour" type of lighting for counting the cell or cells present in the living cell or set of living cells (80) observed by the wide-field camera (121) of the imaging system (120), said "contour" type of lighting being produced using the first lateral source (111a) and the second lateral source (111c), the rays from the first lateral source (111a) and the second lateral source (111c) being split into two collimated beams (113, 114) that are symmetrical with respect to the perpendicular axis (P) to the plane formed by the support (1) of the Petri dish (10) or the Petri dish itself, a first beam (113) from the first lateral source (111a) illuminating the object at an angle of incidence with respect to the perpendicular axis (P) of between 10° and 14°, a second beam (114) from the second lateral source (111c) illuminating the object at an angle of incidence with respect to the perpendicular axis (P) of between 10° and 14°;
- a "relief" type of lighting for viewing the texture and granularity of the cell or cells present in the living cell or set of living cells (80) observed by the wide-field camera (121) of the imaging system (120), said "relief" type of lighting being produced using the first lateral source (111a), a single collimated light beam (115) from the first lateral source (111a) propagating along an axis inclined at an angle with respect to the perpendicular axis (P) of between 8° and 16°;
- means (160) for relative movement of the Petri dish (10) with respect to the assembly formed by the lighting system (110) and said wide-field camera (121) so as to be able to observe living cells or sets of living cells (80) located in the various compartments of the Petri dish (10),
said wide-field camera (121) having a field of view covering at least the total surface area of one of the compartments of the Petri dish (10), and said imaging device (100) being adapted for imaging, without relative movement of said Petri dish with respect to the assembly formed by the lighting system (110) and said wide-field camera (121) using the movement means (160), a living cell or a set of living cells in any position in its compartment in the Petri dish (10) and said wide-field camera (121) having a resolution adapted for observing the details of a living cell or a set of living cells (80), said details having a micrometric size.

2. Imaging device (100) according to claim 1, wherein the set or sets of living cells are one or more embryos.

3. Imaging device (100) according to any one of claims 1 to 2, wherein the movement means (160) are capable of moving the assembly formed by the lighting system (110) and the wide-field camera (121) of the imaging system (120) relatively with respect to the Petri dish (10) in which the living cells or sets of living cells (80) to be observed are deposited.

4. Imaging device (100) according to any one of claims 1 to 3, further comprising a liquid lens (170), adapted for controlling a focal length in the direction of the depth of the living cell or set of living cells.

5. Method for observing the development of living cells or sets of living cells by means of an imaging device (100) as defined in any one of claims 1 to 4, comprising the following steps:
- a preparation step (S300) consisting in successively depositing a living cell or a set of living cells in a Petri dish (10) comprising:
- a container (20) adapted for receiving one or more living cells or sets of living cells to be observed,
- a cover (30),
- an identification element (50) adapted for identifying the living cell or cells or sets of living cells observed,
- specific compartments or wells for depositing a living cell or a set of living cells, said compartments or wells being in the form of cavities (61) adapted for receiving, in addition to the living cell or the set of living cells in the cavity, a drop of a culture medium (62),
followed by a drop of a culture medium (62) in said Petri dish (10), the operation being repeated as many times as necessary depending on the desired number of living cells or sets of living cells to be observed; then covering the whole with a liquid (63), such as oil or water;
- an identification step (S302) for identifying the Petri dish (10) consisting in detecting and viewing, using a dedicated reader, the identification element (50) of the Petri dish (10) in which the living cell or cells or sets of living cells (80) to be observed are located; and
- an observation step (S304) for observing a first living cell or set of living cells (80) using the imaging system (120).

6. Method for observing the development of living cells or sets of living cells according to claim 5, further comprising, prior to the identification step (S302), a step (S301) of approximately positioning the selected Petri dish (10) with respect to the assembly formed by the lighting system (110) and the wide-field camera (121) of the imaging system (120) relative to a Petri dish (10) selected using the movement means, so that the Petri dish (10) is approximately aligned with the assembly formed by the lighting system (110) and the wide-field camera (121) of the imaging system (120).

7. Method for observing the development of living cells or sets of living cells according to claim 5 or 6, comprising, after the step of observing a first living cell or a first set of living cells, a step (S310) of relative movement of the Petri dish (10) with respect to the assembly formed by the lighting system (110) and the wide-field camera (121) of the imaging system (120), making it possible to observe another living cell or another set of living cells located in another compartment (61) of the Petri dish (10).

8. Method for observing the development of living cells or sets of living cells according to any one of claims 5 to 7, in the case where the imaging device (100) further comprises a liquid lens (170), wherein the step of observing a first living cell or a first set of living cells (80) is carried out in a horizontal observation plane parallel to the support (1) for receiving the Petri dish (10) and perpendicular to the direction of the depth of said first living cell or said set of living cells, said plane being determined in an additional determination step (S312) by a configuration of the liquid lens.

9. Method for observing the development of living cells or sets of living cells according to claim 8, wherein the step (S312) of determining an observation plane of said first living cell or set of living cells (80) is repeated for different planes perpendicular to the direction of the depth of said first living cell or set of living cells (80) by means of different configurations of the liquid lens, so as to observe a first living cell or a first set of living cells (80) in the different observation planes determined.
